# EUROPEAN PATENT SPECIFICATION

(11) **EP 3 100 622 B1**
(45) Date of publication and mention of the grant of the patent: **19.10.2022**
(21) Application number: 15170687.6
(22) Date of filing: 04.06.2015
(51) Int. Cl.: A24F 40/42, A24F 40/485, A24F 40/10

(54) **REMOVABLE ATOMIZER AND ELECTRONIC SMOKING DEVICE WITH LATERAL OPENING**
HERAUSNEHMBARER ZERSTÄUBER UND ELEKTRONISCHE RAUCHVORRICHTUNG MIT SEITLICHER ÖFFNUNG
ATOMISEUR AMOVIBLE ET DISPOSITIF À FUMER ÉLECTRONIQUE AVEC OUVERTURE LATÉRALE

(43) Date of publication of application: 07.12.2016
(73) Proprietor: Fontem Holdings 1 B.V., 1083 HN Amsterdam (NL)
(72) Inventor: Yener, Emin, 22761 Hamburg (DE)
(74) Representative: Gulde & Partner

(56) References cited:
- WO-A1-2014/172834
- US-A1- 2014 060 527
- US-A1- 2014 060 554
- US-A1- 2014 123 989
- US-B1- 8 499 766

## Description

### FIELD OF INVENTION

The present invention relates generally to atomizers for electronic smoking devices and to electronic smoking devices, in particular to electronic cigarettes. Atomizers and electronic smoking devices are disclosed in US 2014/0123989 A1, US 2014/0060527 A1, US 2014/00660554 A1, US 8,499,766 B1 and WO 2014/172834 A1.

### BACKGROUND OF THE INVENTION

An electronic smoking device, such as an electronic cigarette (electronic smoking device), typically has a housing accommodating an electric power source (e.g. a single use or rechargeable battery, electrical plug, or other power source), and an electrically operable atomizer. The atomizer vaporizes or atomizes liquid supplied from a reservoir and provides vaporized or atomized liquid as an aerosol. Control electronics control the activation of the atomizer. In some electronic smoking devices, an airflow sensor is provided within the electronic smoking device, which detects a user puffing on the device (e.g., by sensing an under-pressure or an air flow pattern through the device). The airflow sensor indicates or signals the puff to the control electronics to power up the device and generate vapor. In other electronic smoking devices, a switch is used to power up the electronic smoking device to generate a puff of vapor. In case the user of the electronic smoking device wishes to exchange the liquid for another liquid, e.g. a liquid with a different flavor, the new liquid undesirably mixes with the previous liquid in the atomizer, which affects the user's experience.

### SUMMARY OF THE INVENTION

In accordance with one aspect of the invention, there is provided an atomizer for an electronic smoking device. The atomizer comprises a fixing section for removably fixing the atomizer within the electronic smoking device, in particular the electronic smoking device according to an aspect of the invention. Further, the atomizer comprises a housing, wherein the housing has a cylindrical shape with a top base and a bottom base and a side face that interconnects the top and the bottom base. The side face comprises at least one liquid inlet area for receiving liquid to be atomized, and at least one vapour outlet area for releasing atomized liquid. In accordance with another aspect of the invention, there is provided an electronic smoking device comprising a housing and the atomizer according to the invention. The housing is formed with a lateral opening for receiving the atomizer according to the other aspect of the invention. The electronic smoking device comprises a fixing element for fixing the atomizer.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
- Figure 1: is a schematic cross-sectional illustration of an exemplary embodiment of an electronic smoking device;
- Figure 2: shows the exemplary embodiment of Figure 1 in another schematic crosssectional view;
- Figure 3: shows the exemplary embodiment of Figure 1 in an enlarged cross-sectional partial view;
- Figure 4: shows the exemplary embodiment of Figure 1 in a frontal cross-sectional view;
- Figure 5: shows the exemplary embodiment of the atomizer of Figure 4 in a rear view;
- Figure 6: shows the liquid reservoir of the exemplary embodiment of Figure 1 in a front elevation;
- Figure 7: shows another exemplary embodiment of the atomizer of Figure 5 in a rear view;
- Figure 8: shows another exemplary embodiment of the liquid reservoir of Figure 6 in a front elevation; and
- Figures 9 and 10: show the exemplary embodiment of the electronic smoking device of Figure 1 in a top view and in a bottom view, respectively.

### DESCRIPTION OF THE PREFERRED EMBODIMENTS

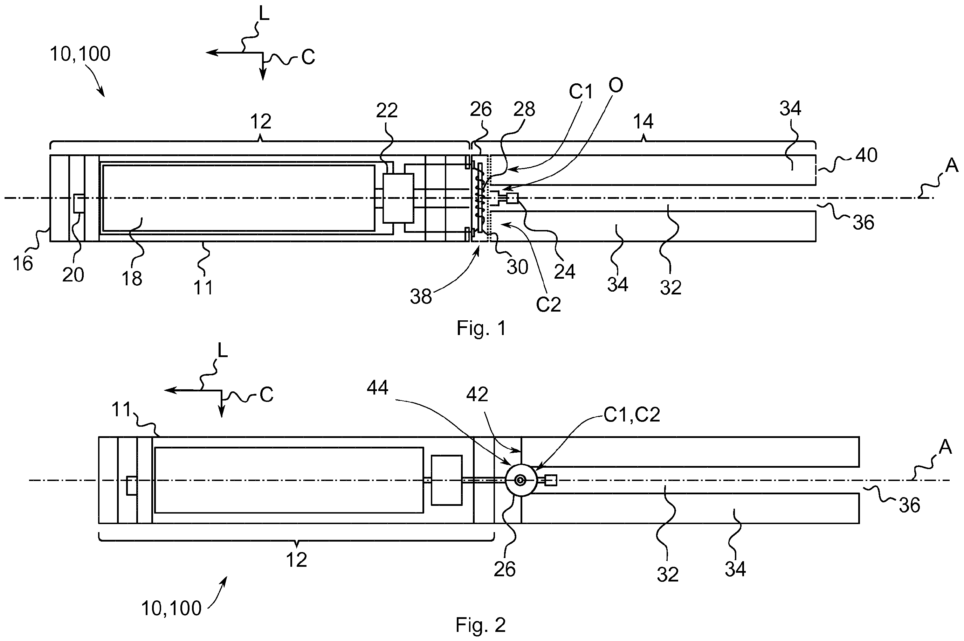

As is shown in Figure 1, an electronic smoking device 10, which may be an e-cigarette, typically has a housing 11 comprising a cylindrical hollow tube having an end cap 16. The cylindrical hollow tube may be a single piece or a multiple piece tube. In Figure 1, the cylindrical hollow tube is shown as a single piece structure having a battery portion 12 and an atomizer/liquid reservoir portion 14. Together, the battery portion 12 and the atomizer/liquid reservoir portion 14 form a cylindrical tube which is approximately the same size and shape as a conventional cigarette, typically about 100 mm with a 7.5 mm diameter, although lengths may range from 70 to 150 or 180 mm, and diameters from 5 to 20 mm. The battery portion 12 and the atomizer/liquid reservoir portion 14 are shown arranged one after the other along a central axis A of the electronic smoking device 10, wherein along the central axis A, a longitudinal direction L of the electronic smoking device 10 extends. Around the central axis A and perpendicular to the longitudinal direction L, a circumferential direction C of the electronic smoking device 10 extends.

The battery portion 12 and atomizer/liquid reservoir portion 14 are typically made of steel or hardwearing plastic and act together with the end cap 16 to provide a housing to contain the components of the electronic smoking device 10. In case the cylindrical hollow tube has a two piece structure with the battery portion 12 and the atomizer/liquid reservoir portion 14, the battery portion 12 and an atomizer/liquid reservoir portion 14 may be configured to fit together by a friction push fit, a snap fit, or a bayonet attachment, magnetic fit, or screw threads. The end cap 16 is provided at the front end of the battery portion 12. The end cap 16 may be made from translucent plastic or other translucent material to allow an LED 20 positioned near the end cap to emit light through the end cap. The end cap can be made of metal or other materials that do not allow light to pass.

A battery 18, the light emitting diode (LED) 20, control electronics 22 and optionally an airflow sensor 24 are provided within the cylindrical hollow tube battery portion 12. The battery 18 is electrically connected to the control electronics 22, which are electrically connected to the LED 20 and the airflow sensor 24. In this example the LED 20 is at the front end of the battery portion 12, adjacent to the end cap 16 and the control electronics 22 and airflow sensor 24 are provided in the central cavity at the other end of the battery 18 adjacent the atomizer/liquid reservoir portion 14.

The airflow sensor 24 acts as a puff detector, detecting a user puffing or sucking on the atomizer/liquid reservoir portion 14 of the electronic smoking device 10. The airflow sensor 24 can be any suitable sensor for detecting changes in airflow or air pressure such a microphone switch including a deformable membrane which is caused to move by variations in air pressure. Alternatively, the sensor may be a Hall element or an electro-mechanical sensor.

The control electronics 22 are also connected to an atomizer 26. In the example shown, the atomizer 26 includes a heating coil 28 which is wrapped around a wick 30 extending across a central passage 32 of the atomizer/liquid reservoir portion 14. The coil 28 may be positioned anywhere in the atomizer 26 and may be perpendicular, transverse or parallel to a liquid reservoir 34 of the electronic smoking device 10. The wick 30 and heating coil 28 do not completely block the central passage 32. Rather, an air gap is provided on either lateral side of the heating coil 28 enabling air to flow past the heating coil 28 and the wick 30. The heating coil 28 comprises a heating wire that is at least section-wise formed as the heating coil 28 and with a plurality of windings.

The atomizer 26 can be removed and may be provided separate from the electronic smoking device 10. An electronic smoking assembly 100, which is ready for usage by a user, comprises the electronic smoking device 10 and the atomizer 26, which may be installed in the electronic smoking device 10.

The central passage 32 is surrounded by a cylindrical liquid reservoir 34 with the ends of the wick 30 abutting or extending into the liquid reservoir 34. The wick 30 may be a porous material such as a bundle of fiberglass fibers, with liquid in the liquid reservoir 34 drawn by capillary action from the ends of the wick 30 towards the central portion of the wick 30 encircled by the heating coil 28.

The liquid reservoir 34 may alternatively include a sponge-like material, e.g. wadding, soaked in liquid, wherein the sponge-like material encircles the central passage 32 with the ends of the wick 30 abutting or extending into the wadding. In other embodiments the liquid reservoir 34 may comprise a toroidal cavity arranged to be filled with liquid and/or the sponge-like material and with the ends of the wick 30 extending into the toroidal cavity.

An air inhalation port 36 is provided at the back end of the atomizer/liquid reservoir portion 14 remote from the end cap 16. The inhalation port 36 may be formed from the cylindrical hollow tube atomizer/liquid reservoir portion 14 or maybe formed in an end cap.

An air inlet may be provided in the end cap 16, at the edge of the inlet next to the cylindrical hollow tube, anywhere along the length of the cylindrical hollow tube, or at the connection of the battery portion 12 and the atomizer/liquid reservoir portion 14. Figure 1 shows that the atomizer 26 is provided with one air inlet 38 at one of its longitudinal ends. The air inlet may be an air inlet opening or an air inlet valve.

In use, a user sucks on the electronic smoking device 10. This causes air to be drawn into the electronic smoking device 10 via one or more air inlets, such as the air inlet 38, and to be drawn through the central passage 32 towards the air inhalation port 36. The change in air pressure which arises is detected by the airflow sensor 24, which generates an electrical signal that is passed to the control electronics 22. In response to the signal, the control electronics 22 activate the heating coil 28, which causes liquid present in the wick 30 to be vaporized creating an aerosol (which may comprise gaseous and liquid components) within the central passage 32. As the user continues to suck on the electronic smoking device 10, this aerosol is drawn through the central passage 32 and inhaled by the user. At the same time, the control electronics 22 also activate the LED 20 causing the LED 20 to light up, which is visible via the translucent end cap 16, mimicking the appearance of a glowing ember at the end of a conventional cigarette. As liquid present in the wick 30 is converted into an aerosol, more liquid is drawn into the wick 30 from the liquid reservoir 34 by capillary action and, thus, is available to be converted into an aerosol through subsequent activation of the heating coil 28.

Some electronic smoking devices are intended to be disposable and the electric power in the battery 18 is intended to be sufficient to vaporize the liquid contained within the liquid reservoir 34; after the battery has been spent, the electronic smoking device 10 is thrown away. In other embodiments, the battery 18 is rechargeable and the liquid reservoir 34 is refillable. In the cases where the liquid reservoir 34 is a toroidal cavity, this may be achieved by refilling the liquid reservoir 34 via a refill port 40. In other embodiments, the liquid reservoir 34 is exchangeable by a replacement liquid reservoir 34 thereby replenishing the supply of liquid.

The replacement liquid reservoir 34 may be in the form of a cartridge having a central passage 32, through which a user inhales aerosol. In other embodiments, aerosol may flow around the exterior of the cartridge 32 to an air inhalation port 36.

Of course, in addition to the above description of the structure and function of the electronic smoking device 10, variations also exist. For example, the LED 20 may be omitted. The airflow sensor 24 may be placed adjacent the end cap 16 rather than in the middle of the electronic smoking device. The airflow sensor 24 may be replaced with a switch, which enables a user to activate the electronic smoking device manually rather than in response to the detection of a change in airflow or air pressure.

In at least one contact area C₁, C₂, the liquid reservoir 34 faces and contacts the atomizer 26 at least section-wise. The atomizer 26 and the liquid reservoir 34 are shown with dotted lines in the at least one contact area C₁, C₂, which indicate that the atomizer 26 and the liquid reservoir 34 are permeable for liquid stored in the liquid reservoir 34 in the at least one contact area C₁, C₂. In particular, a side face of the atomizer 26 contacts a front face of the liquid reservoir 34 at least section-wise, wherein the side face and front face are each at least partly permeable for liquid stored in the liquid reservoir 34. The side face of the atomizer 26 faces the liquid reservoir 34 and contacts the liquid reservoir 34 in the at least one contact area C₁, C₂.

The atomizer 26 and the liquid reservoir 34 may contact each other in at least two contact areas C₁, C₂, wherein the central passage 32 is arranged between the at least two contact areas C₁, C₂, for example perpendicular to the central axis A.

Between the contact areas C₁, C₂, which may be arranged one after the other perpendicular to a central axis A of the electronic smoking device 10, the atomizer 26 is formed with at least one vapor outlet area O that is in communication with the central passage 32.

Different types of atomizers 26 may be used. Thus, for example, the atomizer may 26 has the heating coil 28 in a cavity in the interior of a porous body, e.g. the wick 30, soaked in liquid to ne atomized. In this design aerosol is generated by evaporating the liquid within the porous body either by activation of the coil 28 heating the porous body or alternatively by heated air passing over or through the porous body. Alternatively, the atomizer 26 may be wickless or may for example be adapted to atomize liquid by ultrasound.

Figure 2 shows the exemplary embodiment of the electronic smoking assembly 100 with the electronic smoking device 10 and the atomizer 26 of Figure 1 in another cross-sectional side view schematically, wherein the electronic smoking device 10 is shown rotated around a central axis A of the electronic smoking device 10 by 90°.

As shown in Figure 2, the front face 42 of the liquid reservoir 34 is formed with a groove 44 that opens towards the battery portion 12 and/or away from the air inhalation port 36. The atomizer 26 is at least section-wise arranged in the groove 44, which may decrease the total length of the electronic smoking device 10 along its central axis A and increase the surface of the contact areas C₁, C₂. In particular, the contact areas C₁, C₂ are arranged in the groove 44 and for example at a bottom of the groove 44.

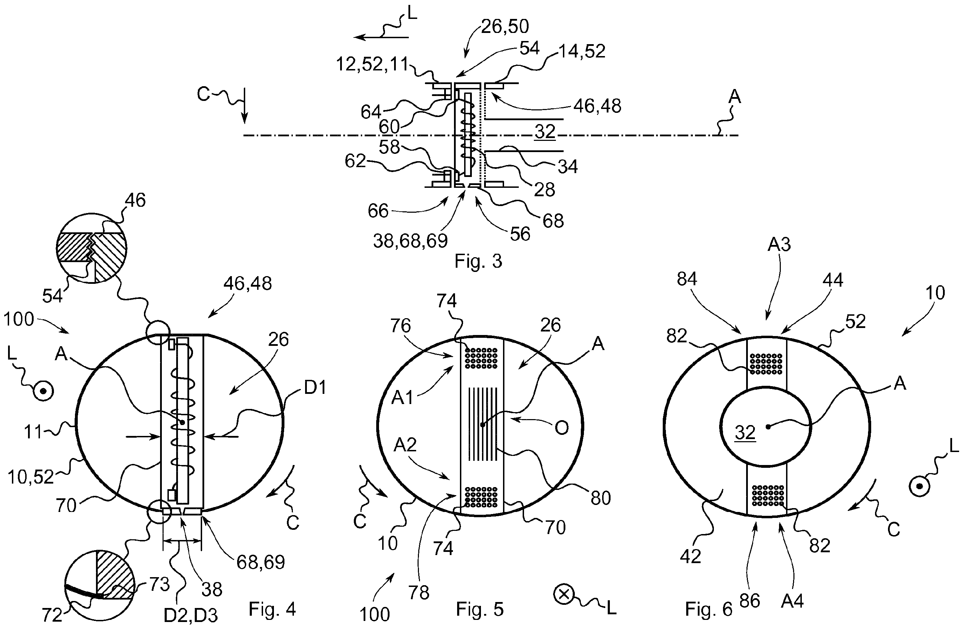

Figure 3 shows the exemplary embodiment of the electronic smoking assembly 100 with the electronic smoking device 10 and the atomizer 26 shown in Figure 1 partly in an enlarged cross-sectional view as shown in Fig. 1.

The atomizer 26 is formed with a fixing section 46 for removably fixing the atomizer 26 within the electronic smoking device 10. The fixing section 46 is arranged at a longitudinal end of the atomizer 26, which is designated as a fixing end 48 of the atomizer 26. For example, the fixing section 46 may comprise an outside fixing thread for affixing the atomizer 26 to the electronic smoking device 10 by a screw connection. Alternatively, the fixing section 46 may be adapted to form another force fit or a form fit with the electronic smoking device 10. For example, the fixing section 46 may be adapted to form a press fit or a bayonet connection with the electronic smoking device 10.

The atomizer 26 is inserted into the electronic smoking device 10 via a lateral opening 50 of the electronic smoking device 10. The lateral opening 50 may be formed by an external housing 52 of the electronic smoking device 10. In order to affix the atomizer 26 to the electronic smoking device 10, the electronic smoking device 10 comprises a fixing element 54 for fixing the atomizer 26. The fixing element 54 is a counter fixing element for the fixing section 46 and is adapted to interact with the fixing section 46 in order to fix the atomizer 26 to the electronic smoking device 10 and e.g. to its housing 52. For example, the fixing element 54 may be adapted to form a force fit, e.g. a press fit, or a form fit, e.g. a bayonet connection, with the fixing section 46 of the atomizer 26. In case the fixing section 46 is formed with the outside thread, the fixing element 54 may be formed as an internal thread.

The lateral opening 50 borders on the fixing element 54, such that the fixing element 54 delimits the lateral opening 50 along and perpendicular to the central axis A, for example along the longitudinal direction L and the circumferential direction C of the electronic smoking device 10. For example, the fixing element 54 comprises the internal thread that forms an edge of the lateral opening 50. The internal thread may extend around the lateral opening 50 and may be delimited by the edge of the housing 52 that borders on the lateral opening 50.

Within the electronic smoking device 10 and in particular within the housing 52, a receiving volume 56 for the atomizer 26 is arranged, wherein the receiving volume 56 is at least section-wise essentially complementary to an outer shape of the atomizer 26. Hence, the liquid reservoir 34 and in particular its front face with the contact areas C₁, C₂ borders on the receiving volume 56. Via the lateral opening 50, the receiving volume 56 is readily accessible for the outside of the electronic smoking device 10 for insertion of the atomizer 26. When inserted into the receiving volume 56, the atomizer may not protrude from the housing 52 and may be flush with a lateral outer surface of the housing 52.

The atomizer 26 comprises two contact elements 58, 60 for connecting the heating coil 28 with the power supply and for example with the battery 18. The electronic smoking device 10 comprises two counter contact elements 62, 64 for connecting the contact elements 58, 60. The counter contact elements 62, 64 are connected to the power supply, e.g. the battery 18, via the control electronics 22. For example, the contact elements 58, 60 may be brought into mechanical contact with the counter contact elements 62, 64 by inserting the atomizer 26 into the receiving volume 56 perpendicular to the central axis A. Hence, the counter contact elements 62, 64 border on the receiving volume 56 opposite of the liquid reservoir 34. Alternatively, the atomizer 26 may be rotated and e.g. screwed after at least partially inserting the atomizer 26 into the receiving volume 56 in order to bring the contact elements 58, 60 in contact with the counter contact elements 62, 64.

In addition to the lateral opening 50, the electronic smoking device 10 of the exemplary embodiment of Figure 3 comprises a second lateral opening 66, wherein the lateral openings 50, 66 are arranged opposite of each other with the central axis A therebetween. Hence, the lateral openings 50, 66 are arranged one after the other perpendicular to the central axis A. In case the lateral opening 50 borders on the fixing element 54, the other lateral opening 66 may receiving an air inlet section 68 of the atomizer 26, wherein the air inlet section 68 may be the longitudinal air inlet end 69 of the atomizer 26 opposite of its fixing end 48. The air inlet section 68 comprises at least one air inlet that is in communication with the outside of the electronic smoking device 10 when the atomizer 26 is mounted to the electronic smoking device 10 and in particular when the atomizer 26 is inserted into the receiving volume 56.

The two lateral openings 50, 66 may have identical inner diameters. The receiving volume 56 extends from one of the lateral openings 50 to the other one of the lateral openings 66, wherein at least one of the lateral openings 50, 66 may be part of the receiving volume 56.

Alternatively, the fixing section 46 and the air inlet section 68 may at least sectionwise or even completely overlap one another and may be arranged at the same longitudinal end of the atomizer 26. In this case, only one of the lateral openings 50, 66 may be necessary.

Figure 4 shows the electronic smoking assembly 100 with the electronic smoking device 10 and the atomizer 26 of the exemplary embodiment of Figure 1 in another enlarged cross-sectional view, wherein the central axis A extends perpendicular to the plane of projection.

The fixing section 46 and the fixing element 54 are shown with threads, which provide that the atomizer 26 is mountable to the electronic smoking device 10 by a screw connection. Between the fixing end 48 and the air inlet end 68, the atomizer 26 is formed essentially cylindrically, e.g. as a right circular cylinder. In particular, between the fixing end 48 and the air inlet end 68, the atomizer 26 is formed with a housing 70, which may be formed cylindrically and in particular as a right circular cylinder, e.g. as a sleeve. Perpendicular and/or parallel to the central axis A, the housing 70 comprises an outer diameter D₁. The air inlet end 69 is formed with an outer diameter D₂, wherein the outer diameter D₂ is smaller than the outer diameter D₁ according to the exemplary embodiment of Figure 4. Hence, at the air inlet end 69, a front face 72 of the housing 70 forms a stop element.

An inner diameter D₃ of the lateral opening 66 is dimensioned to be between the outer diameter D₁ of the housing 70 and the outer diameter D₂ of the air inlet end 69, such that it forms a counter stop element 73 for the front face 72. In particular, an edge of the housing 52 of the electronic smoking device 10 that borders on the lateral opening 66 forms the counter stop element 73 and engages into a groove that is formed at the air inlet end 69 by the protruding front face 72, the groove opening away from the central axis A and in the circumferential direction C.

An end plate of the atomizer 26 that comprises the at least one air inlet 38 is shown arranged in the lateral opening 66 and can be flush with the outer lateral surface of the housing 52 of the electronic smoking device 10 when the atomizer 26 is mounted to the electronic smoking device 10.

Figure 5 shows the electronic smoking assembly 100 with the electronic smoking device 10 and the atomizer 26 of the exemplary embodiment of Figure 1 in an enlarged cross-sectional view opposite of the view of Figure 4.

The housing 70 of the atomizer 26 is formed with at least one or even two or more liquid inlet areas A₁, A₂ that are adapted to be permeable for liquid received from the liquid reservoir 34. In order to be permeable for liquid received from the liquid reservoir 34, the two liquid inlet areas A₁, A₂ are exemplarily shown with a plurality of liquid inlet openings 74 formed as inlet holes. Due to the liquid inlet areas A₁, A₂, liquid to be atomized can enter the atomizer 26 and can be transported to the heating coil 28 by the wick 30. For example, the wick 30 is in close contact with at least one or even all of the liquid inlet areas A₁, A₂ in order to receive the liquid to be atomized. The at least two liquid inlet areas A₁, A₂, are arranged at a distance from each other perpendicular to the central axis A. The plurality of liquid inlet openings 74 is grouped into groups 76, 78, the two groups 76, 78 being arranged at a distance from each other perpendicular to the central axis A.

The housing 70 of the atomizer 26 is formed with at least one vapor outlet area O that are adapted to be permeable for atomized or vaporized liquid and to let the atomized or vaporized liquid exit the atomizer 26. The at least one vapor outlet area O is exemplarily shown arranged between the two liquid inlet areas A₁, A₂.

In order to be permeable for atomized or vaporized liquid, at least one vapor outlet area O may comprise at least one vapor outlet opening 80, via which atomized liquid can escape the atomizer 26. The at least one vapor outlet opening 80 is formed as a longitudinal slot. For example, the slot-shaped vapor outlet opening 80 may extend perpendicular to the central axis A. A plurality of vapor outlet openings 80 may be provided. Several of each of the vapor outlet openings 80 may extend parallel to at least one other of the vapor outlet openings 80. For example, all vapor outlet openings 80 may extend from the group 76 towards the group 78 of liquid inlet openings 74. Furthermore, each of the vapor outlet openings 80 may be arranged at a distance to each of the groups 74, 78.

Figure 6 shows the liquid reservoir 34 arranged in the housing 52 of the electronic smoking device 10 in a frontal view against the longitudinal direction L.

The groove 44 extends perpendicular to the central axis A and against the longitudinal direction L through the front face 42 of the liquid reservoir 34. Hence, the groove 44 opens in or against the longitudinal direction L. Within the groove 44, the liquid reservoir 34 is formed with at least one, two or more liquid outlet areas A₃, A₄. The at least one liquid outlet area A₃, A₄ is permeable for fluid to be atomized stored in the liquid reservoir 34. In order to be permeable for fluid to be atomized stored in the liquid reservoir 34, the at least one liquid outlet area A₃, A₄ may comprise at least one liquid outlet opening 82. The at least one liquid outlet opening 82 is adapted to issue liquid to be atomized to the atomizer 26.

The liquid reservoir 34 is shown with two liquid outlet areas A₃, A₄, each comprising a plurality of liquid outlet openings 82, wherein the liquid outlet openings 82 are arranged in two groups 84, 86 within the groove 44. Between the two liquid outlet areas A₃, A₄, the central passage 32 extends along the central axis A.

In a ready-for-use state of the electronic smoking assembly 100, in which the atomizer 26 is mounted to the electronic smoking device 10, the at least one liquid outlet area A₃ at least section-wise overlaps the at least one liquid inlet area A_{1.} In case two or more liquid outlet areas A₃, A₄ and two or more liquid inlet areas A₁, A₂ are provided, each of the liquid outlet areas A₃, A₄ may at least sectionwise or even completely overlap at least one of the liquid inlet areas A₁, A₂.

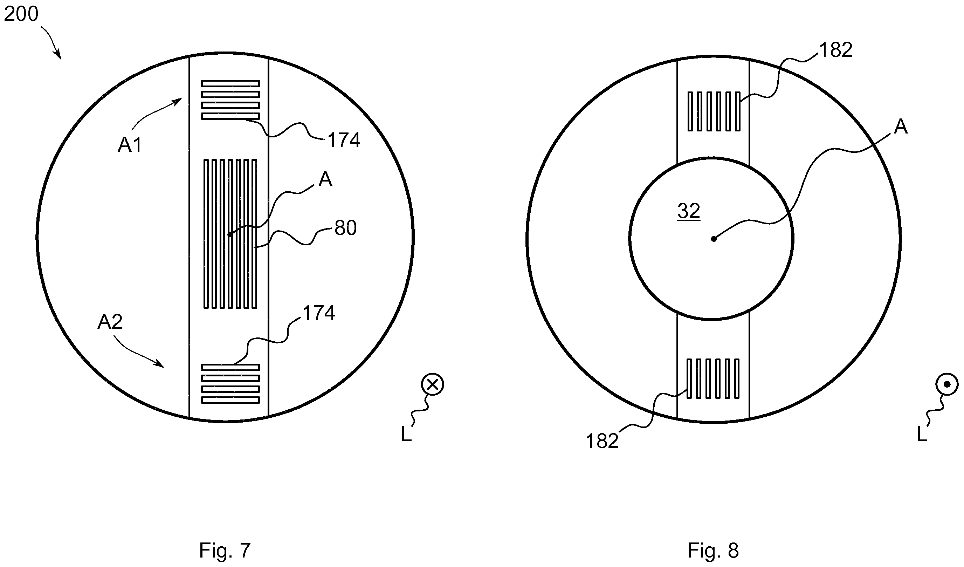

Figure 7 shows another exemplary embodiment of the electronic smoking assembly 200 with the electronic smoking device 210 and the atomizer 126 shown in Figure 5. For the sake of brevity, only the differences from the exemplary embodiment of Figure 5 are looked at.

The liquid inlet openings 174 are formed with a length that is greater than a width perpendicular to the length of the openings. At least two or all of the elongated or oblong liquid inlet openings 174 are arranged parallel to each other and for example perpendicular to the at least one vapor outlet opening 80. In particular, as exemplarily shown in Figure 7, the liquid inlet openings 174 are formed stripe- or slit-shaped.

Figure 8 shows another exemplary embodiment of the liquid reservoir 134 arranged in the housing 52 of the electronic smoking device 110 in a frontal view against the longitudinal direction L. For the sake of brevity, only the differences from the exemplary embodiment of Figure 5 are looked at.

The liquid outlet openings 182 are formed with a length that is greater than a width perpendicular to the length of the openings. At least two or all of the elongated or oblong liquid outlet openings 182 are arranged parallel to each other and for example perpendicular to the at least one vapor outlet opening 80 and/or at least one or all of the liquid inlet openings 174. In particular, as exemplarily shown in Figure 7, the liquid outlet openings 182 are formed stripe- or slit-shaped such that they can extend away from the central axis 10 and/or the central passage 32.

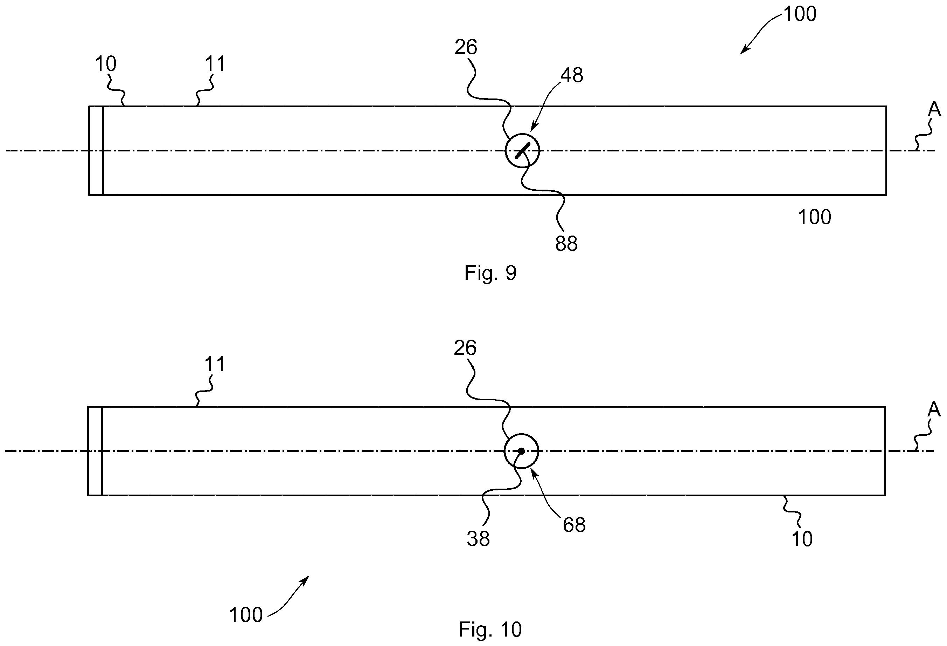

Figures 9 and 10 schematically show the electronic smoking assembly 100, 200 of the exemplary embodiment of the previous figures in a top and a bottom view. The electronic smoking device 10, 110 accommodates the atomizer 26, 126, such that the electronic smoking assembly 100, 200 is shown in a ready-to-use state in Figures 9 and 10.

Figure 9 shows the electronic smoking assembly 100, 200 with the fixing end 48 of the atomizer 26, 126 facing out of the plane of projection. The fixing end 48 comprises a recess 88, into which a tool, for example a screwdriver or a coin, or a fingernail of the user, can be inserted in order to be able to turn the atomizer 26, 126, e.g. in case the atomizer 26, 126 can be affixed to the electronic smoking device 10, 110 by screwing. Figure 10 shows the electronic smoking assembly 100, 200 with the air inlet end 48 facing away from the plane of projection.

It will also be appreciated that although in the above-explained embodiments a puff detector (utilizing an airflow sensor) for detecting a user puffing on a device could be provided and the puff detector could be arranged to initiate the activation of an atomizer when a user puffed on the device, in some embodiments, the puff detector could be replaced by a push button and a user could cause an atomizer to activate by pressing on the button. In other embodiments, other devices for activating the device could be provided.

In summary, the atomizer for an electronic smoking device comprises a fixing section for removably fixing the atomizer within the electronic smoking device. Hence, one advantage of such an atomizer may be that the atomizer can be removed from the electronic smoking device in case the electronic smoking device is e.g. used with a different liquid, which shall not mix with the liquid already present in the atomizer from a previous usage of the electronic smoking device with the atomizer.

Moreover, the electronic smoking device comprises a housing, wherein the housing is formed with a lateral opening for receiving the atomizer, and wherein the electronic smoking device comprises a fixing element for fixing the atomizer. One advantage of this aspect may be that, due to the lateral opening, the atomizer can be easily mounted to and removed from the electronic smoking device. Another advantage of this aspect may be that the atomizer can be readily affixed when mounted to the electronic smoking device without the need of additional and for example separate fixing elements. For example, the fixing section and the fixing element may be adapted to form a form fit, e.g. a bayonet connection. Alternatively, the fixing element and the fixing section may be adapted to form a force fit, for example a press fit or a screw connection.

The fixing section may be arranged at a fixing end of the atomizer. Thus, an advantage of this aspect may be that the fixing section does not interfere with liquid and vapor provision and thus does not affect the smoking experience of the user.

The lateral opening may border on the fixing element. An advantage of this aspect may be that the fixing element does not require additional space and that it can readily interact with the fixing end of the atomizer.

The fixing section may comprise an outside fixing thread. This aspect may have the advantage that the atomizer can readily be fixed and removed without wearing the electronic smoking device due to e.g. a press fit.

The fixing element of the electronic smoking device may comprise an internal thread that forms an edge of the lateral opening. Hence, the advantage of this aspect may be that the atomizer with the external thread can be readily connected.

The fixing section may be circular and the internal thread may extend around the lateral opening in order to facilitate screwing. The internal thread may be formed by the edge of the housing of the electronic smoking device that borders on and restricts the lateral opening, e.g. along a longitudinal direction and a circumferential direction of the electronic smoking device.

The atomizer may comprise an air inlet section at one of its ends. Thus, an advantage of this aspect may be that the electronic smoking device does not need to be formed with a separate air inlet, which may facilitate producibility of the electronic smoking device. The air inlet section may comprise an air inlet opening and/or an air inlet valve, wherein the air inlet valve may be adapted to let air enter the atomizer and to prevent that liquid within the atomizer flows out of the atomizer through the air inlet valve.

In case one air inlet section is sufficient, the electronic smoking device may be formed without any additional air inlets, e.g. air inlet openings or valves.

The air inlet section may be arranged at the one of the ends of the atomizer that is opposite of its fixing end. The ends of the atomizer can be longitudinal ends that are arranged opposite of each other and/or facie away from each other. One advantage of the arrangement of the air inlet section at one of the longitudinal ends of the atomizer may be that the atomizer can communicate with the environment of the electronic smoking device directly and without the need to let air stream through the electronic smoking device to the atomizer.

The electronic smoking device may comprise a liquid reservoir for liquid to be atomized, and a receiving volume for an atomizer, wherein the liquid reservoir borders on the receiving volume. An advantage of this aspect may be that installation space for the atomizer and for the liquid reservoir is efficiently used, such that a maximum of liquid can be stored within the electronic smoking device.

The form of the receiving volume may be essentially complementary to the form of the atomizer. For example, the form of the receiving volume may correspond to a cylinder, e.g. a right cylinder, or a right circular cylinder, with the lateral opening as footprint.

The liquid reservoir may comprise at least one liquid outlet area, wherein the liquid outlet area is permeable for liquid. An advantage of this aspect may be that liquid to be atomized or vaporized can be easily ejected from the liquid reservoir without the need of separate liquid ducts.

The liquid reservoir may be formed with a groove, for example in its front face, in which the receiving volume is at least sectionwise arranged. The groove may be formed essentially complementary to a section of the atomizer, such that the atomizer can be inserted into the groove at least partly, thereby possibly reducing to total size of the electronic smoking device and increasing a contact area between the liquid reservoir and the atomizer.

The atomizer comprises a housing and an atomizing element, wherein the atomizing element may be arranged in the housing. The housing has a cylindrical shape and may be a hollow right cylinder or a hollow right circular cylinder.

The atomizer's housing comprises at least one liquid inlet area for receiving liquid to be atomized. Hence, again, due to the liquid inlet area, no complicated liquid duct system needs to be installed and connected to the atomizer when mounting the atomizer.

The housing of the atomizer comprises at least one vapor outlet area for releasing atomized liquid. Hence, an advantage of this aspect may be that vaporized liquid can easily escape the atomizer and can be provided to the user with a minimum of condensation of atomized liquid, which improves the user's experience.

The liquid reservoir may comprise at least one or at least two liquid outlet areas and a gas duct. The gas duct is for example a central passage for receiving atomized liquid, e.g. mixed with air, wherein the gas duct is arranged between the liquid outlet openings. Hence, an advantage of this aspect may be that known electronic smoking devices with liquid reservoirs having a gas duct, e.g. the central passage, can be used with the liquid reservoir according to at least one aspect. Furthermore, another advantage of this aspect may be that, due to the provision of two liquid outlet areas, the amount of liquid to be atomized provided to the atomizer can be maximized, thereby providing an improved user experience.

The liquid inlet openings may be formed as circular liquid inlet openings or with a length that is greater than a width perpendicular to the length of the openings. At least two or all of the elongated or oblong liquid inlet openings can be arranged parallel to each other and for example perpendicular to the at least one vapor outlet opening. In particular, for example, the liquid inlet openings are formed stripe- or slit-shaped.

The liquid outlet openings may be formed with a length that is greater than a width perpendicular to the length of the openings. At least two or all of the elongated or oblong liquid outlet openings can be arranged parallel to each other and for example perpendicular to the at least one vapor outlet opening and/or at least one or all of the liquid inlet openings. In particular, for example, the liquid outlet openings are formed stripe- or slit-shaped such that they can extend away from the central axis and/or the central passage.

Elongated or oblong liquid inlet and liquid outlet openings with different orientations may have the advantage that even with positional tolerances of the openings, the liquid inlet openings can be brought in overlap with the liquid outlet openings, thereby ensuring that liquid can flow from the liquid reservoir into the atomizer and facilitating producability of the electronic smoking device and the atomizer. For example, the liquid inlet and liquid outlet openings cross each other and, therefore, overlap each other even in case of positional tolerances between the atomizer and the liquid reservoir.

The liquid outlet areas may be arranged in the groove of the liquid reservoir, in order to possibly increase the contact area between the at least one liquid outlet area and the at least one liquid inlet area. The aspect may have the advantage that liquid can be supplied to the atomizer with a high flow rate, such that more vapor can be produced during one puff, which improves the user's experience.

The housing of the atomizer may comprise at least two liquid inlet areas. In case the housing of the atomizer comprises more than one liquid inlet area, the vapor outlet area can be arranged between the liquid inlet areas. One advantage of this aspect may be that, compared to an atomizer with only one liquid inlet area, the amount of liquid to be atomized can be maximized, in particular in combination with the liquid reservoir of the above-mentioned aspect.

The housing of the electronic smoking device may comprise two lateral openings that are arranged opposite of each other, wherein e.g. only one of the two letter openings borders on the fixing element. The other one may receive the air inlet end of the atomizer. One advantage of this aspect may be that the atomizer can be securely affixed without risking to contaminate the air inlet end by affixing the atomizer.

The two lateral openings may have essentially identical or different inner diameters. In particular, the lateral opening receiving the air inlet ends of the atomizer at least section-wise may have an inner diameter that is smaller than a maximum outer diameter of the atomizer. The other lateral opening may have an inner diameter that is larger than the inner diameter of the lateral opening receiving the air inlet end. The lateral opening with the larger inner diameter may be adapted to fix the fixing section. The lateral opening receiving the air inlet section may form a stop for the atomizer such that the atomizer cannot unintentionally fall through the two lateral openings and out of the electronic smoking device.

The receiving volume may extend from one of the lateral openings to the other one of the lateral openings, wherein at least one of the lateral openings is part of the receiving volume. Hence, an advantage of this aspect may be that the atomizer can be easily inserted into the receiving volume via one of the lateral openings.

### LIST OF REFERENCE SIGNS

- 10, 110: electronic smoking device
- 11: housing
- 12: battery portion
- 14: atomizer/liquid reservoir portion
- 16: end cap
- 18: battery
- 20: light emitting diode (LED)
- 22: control electronics
- 24: airflow sensor
- 26, 126: atomizer
- 28: heating coil
- 30: wick
- 32: central passage
- 34, 134: liquid reservoir
- 36: air inhalation port
- 38: air inlets
- 40: refill port
- 42: front face of 34
- 44: groove in 34
- 46: fixing section of 26
- 48: fixing end of 26
- 50, 66: lateral opening of 10
- 52: housing of 10
- 54: fixing element of 10
- 56: receiving volume
- 58, 60: contact element
- 62, 64: counter contact element
- 68: air inlet section
- 69: air inlet end
- 70: housing or 26
- 72: front face of 70
- 73: counter stop element
- 74, 174: liquid inlet opening
- 76, 78: group of 74
- 80: vapor outlet opening
- 82, 182: liquid outlet opening
- 84, 86: group of 82
- 88: recess
- 100, 200: electronic smoking assembly

- A: central axis of 10
- A₁, A₂: liquid inlet area
- A₃, A₄: liquid outlet area
- C: circumferential direction
- C₁, C₂: contact areas
- D₁: outer diameter of 70
- D₂: outer diameter of 69
- D₃: inner diameter of 66
- L: longitudinal direction
- O: vapor outlet area

## Claims

1. Atomizer (26) for an electronic smoking device (10), wherein the atomizer (26) comprises a fixing section (46) for removably fixing the atomizer (26) within the electronic smoking device (10), wherein the atomizer (26) comprises a housing (70), wherein the housing (70) has a cylindrical shape with a top base and a bottom base and a side face that interconnects the top and the bottom base, **characterized in that** the side face comprises at least one liquid inlet area (A₁, A₂) for receiving liquid to be atomized, and at least one vapor outlet area (O) for releasing atomized liquid.

2. Atomizer (26) according to claim 1, **characterized in that** the fixing section (46) is arranged at a fixing end (48) of the atomizer (26).

3. Atomizer (26) according to claim 1 or 2, **characterized in that** the fixing section (46) comprises an outside fixing thread.

4. Atomizer (26) according to any of claims 1 to 3, **characterized in that** the atomizer (26) comprises an air inlet section (68) at one of its ends (48, 69).

5. Atomizer (26) according to claim 4, **characterized in that** the air inlet section (68) is arranged at the one of the longitudinal ends (69) of the atomizer (26) that is opposite of the fixing end (48).

6. Atomizer (26) according to claim 1, **characterized in that** the housing (70) comprises at least two liquid inlet areas (A₁, A₂), wherein the vapor outlet area (O) is arranged between the at least two liquid inlet areas (A₁, A₂).

7. Electronic smoking device (10) comprising a housing (52), wherein the housing (52) is formed with a lateral opening (50, 66) for receiving an atomizer (26), and wherein the electronic smoking device (10) comprises a fixing element (54) for fixing the atomizer (26), and an atomizer (26) inserted into the electronic smoking device (10) via the lateral opening (50, 66), **characterized in that** the atomizer (26) is the atomizer (26) of any of claims 1 to 6.

8. Electronic smoking device (10) according to claim 7, **characterized in that** the lateral opening (50) borders on the fixing element (54).

9. Electronic smoking device (10) according to claim 7 or 8, **characterized in that** the fixing element (54) comprises an internal thread that forms an edge of the lateral opening (50).

10. Electronic smoking device (10) according to any of claims 7 to 9, **characterized in that** the electronic smoking device (10) comprises a liquid reservoir (34) for liquid to be atomized, and a receiving volume (56) for the atomizer (26), wherein the liquid reservoir (34) borders on the receiving volume (56).

11. Electronic smoking device (10) according to claim 10, **characterized in that** the liquid reservoir (34) is formed with a groove (44), in which the receiving volume (56) is at least section-wise arranged.

12. Electronic smoking device (10) according to claim 10 or 11, **characterized in that** the liquid reservoir (34) comprises at least one liquid outlet area (A₃, A₄), wherein the liquid outlet area (A₃, A₄) is permeable for liquid.

13. Electronic smoking device (10) according to claim 12, **characterized in that** the liquid reservoir (34) comprises at least two liquid outlet areas (A₃, A₄) and a central passage (32) for receiving atomized liquid, wherein the central passage (32) is arranged between the at least two liquid outlet areas (A₃, A₄).

## Patentansprüche

1. Verdampfer (26) für eine elektronische Rauchvorrichtung (10), wobei der Verdampfer (26) einen Befestigungsabschnitt (46) zum lösbaren Befestigen des Verdampfers (26) innerhalb der elektronischen Rauchvorrichtung (10) aufweist, wobei der Verdampfer (26) ein Gehäuse (70) aufweist, wobei das Gehäuse (70) eine zylindrische Form mit einer oberen Grundfläche und einer unteren Grundfläche und einer Seitenfläche, welche die obere und die untere Grundfläche miteinander verbindet, aufweist, **dadurch gekennzeichnet, dass** die Seitenfläche wenigstens einen Liquideinlassbereich (A₁, A₂) zum Aufnehmen eines zu verdampfenden Liquids und wenigstens einen Dampfauslassbereich (O) zum Abgeben von verdampftem Liquid aufweist.

2. Verdampfer (26) nach Anspruch 1, **dadurch gekennzeichnet, dass** der Befestigungsabschnitt (46) an einem Befestigungsende (48) des Verdampfers (26) angeordnet ist.

3. Verdampfer (26) nach Anspruch 1 oder 2, **dadurch gekennzeichnet, dass** der Befestigungsabschnitt (46) ein äußeres Befestigungsgewinde aufweist.

4. Verdampfer (26) nach einem der Ansprüche 1 bis 3, **dadurch gekennzeichnet, dass** der Verdampfer (26) an einem seiner Enden (48, 69) einen Lufteinlassabschnitt (68) aufweist.

5. Verdampfer (26) nach Anspruch 4, **dadurch gekennzeichnet, dass** der Lufteinlassabschnitt (68) an dem einen der Längsenden (69) des Verdampfers (26), das dem Befestigungsende (48) gegenüberliegt, angeordnet ist.

6. Verdampfer (26) nach Anspruch 1, **dadurch gekennzeichnet, dass** das Gehäuse (70) wenigstens zwei Liquideinlassbereiche (A₁, A₂) aufweist, wobei der Dampfauslassbereich (O) zwischen den wenigstens zwei Liquideinlassbereichen (A₁, A₂) angeordnet ist.

7. Elektronische Rauchvorrichtung (10), die ein Gehäuse (52) aufweist, wobei das Gehäuse (52) mit einer seitlichen Öffnung (50, 66) zum Aufnehmen eines Verdampfers (26) ausgebildet ist und wobei die elektronische Rauchvorrichtung (10) ein Befestigungselement (54) zum Befestigen des Verdampfers (26) und einen Verdampfer (26), der über die seitliche Öffnung (50, 66) in die elektronische Rauchvorrichtung (10) eingesetzt ist, aufweist, **dadurch gekennzeichnet, dass** der Verdampfer (26) der Verdampfer (26) nach einem der Ansprüche 1 bis 6 ist.

8. Elektronische Rauchvorrichtung (10) nach Anspruch 7, **dadurch gekennzeichnet, dass** die seitliche Öffnung (50) an das Befestigungselement (54) angrenzt.

9. Elektronische Rauchvorrichtung (10) nach Anspruch 7 oder 8, **dadurch gekennzeichnet, dass** das Befestigungselement (54) ein Innengewinde aufweist, das einen Rand der seitlichen Öffnung (50) ausbildet.

10. Elektronische Rauchvorrichtung (10) nach einem der Ansprüche 7 bis 9, **dadurch gekennzeichnet, dass** die elektronische Rauchvorrichtung (10) ein Liquidreservoir (34) für zu verdampfendes Liquid und ein Aufnahmevolumen (56) für den Verdampfer (26) aufweist, wobei das Liquidreservoir (34) an das Aufnahmevolumen (56) angrenzt.

11. Elektronische Rauchvorrichtung (10) nach Anspruch 10, **dadurch gekennzeichnet, dass** das Liquidreservoir (34) mit einer Nut (44) ausgebildet ist, in der wenigstens abschnittsweise das Aufnahmevolumen (56) angeordnet ist.

12. Elektronische Rauchvorrichtung (10) nach Anspruch 10 oder 11, **dadurch gekennzeichnet, dass** das Liquidreservoir (34) wenigstens einen Liquidauslassbereich (A₃, A₄) umfasst, wobei der Liquidauslassbereich (A₃, A₄) für Liquid durchlässig ist.

13. Elektronische Rauchvorrichtung (10) nach Anspruch 12, **dadurch gekennzeichnet, dass** das Liquidreservoir (34) wenigstens zwei Liquidauslassbereiche (A₃, A₄) und einen mittigen Durchlass (32) zum Aufnehmen von verdampftem Liquid umfasst, wobei der mittige Durchlass (32) zwischen den wenigstens zwei Liquidauslassbereichen (A₃, A₄) angeordnet ist.

## Revendications

1. Atomiseur (26) pour un dispositif de cigarette électronique (10), l'atomiseur (26) comprenant une section de fixation (46) pour fixer de façon amovible l'atomiseur (26) dans le dispositif de cigarette électronique (10), l'atomiseur (26) comprenant un boîtier (70), le boîtier (70) ayant une forme cylindrique avec une base supérieure et une base inférieure et une face latérale qui relie la base supérieure et la base inférieure, **caractérisé en ce que** la face latérale comprend au moins une zone d'admission de liquide (A₁, A₂) pour recevoir du liquide devant être pulvérisé, et au moins une zone de sortie de vapeur (O) pour libérer du liquide pulvérisé.

2. Atomiseur (26) selon la revendication 1, **caractérisé en ce que** la section de fixation (46) est disposée sur une extrémité de fixation (48) de l'atomiseur (26).

3. Atomiseur (26) selon la revendication 1 ou 2, **caractérisé en ce que** la section de fixation (46) comprend un filetage de fixation extérieur.

4. Atomiseur (26) selon l'une quelconque des revendications 1 à 3, **caractérisé en ce que** l'atomiseur (26) comprend une section d'admission d'air (68) à l'une de ses extrémités (48, 69).

5. Atomiseur (26) selon la revendication 4, **caractérisé en ce que** la section d'admission d'air (68) est disposée sur celle des extrémités longitudinales (69) de l'atomiseur (26) qui est opposée à l'extrémité de fixation (48).

6. Atomiseur (26) selon la revendication 1, **caractérisé en ce que** le boîtier (70) comprend au moins deux zones d'admission de liquide (A₁, A₂), la zone de sortie de vapeur (O) étant disposée entre les au moins deux zones d'admission de liquide (A₁, A₂).

7. Dispositif de cigarette électronique (10) comprenant un boîtier (52), le boîtier (52) étant formé avec une ouverture latérale (50, 66) pour recevoir un atomiseur (26), et le dispositif de cigarette électronique (10) comprenant un élément de fixation (54) pour fixer l'atomiseur (26), et un atomiseur (26) inséré dans le dispositif de cigarette électronique (10) via l'ouverture latérale (50, 66), **caractérisé en ce que** l'atomiseur (26) est l'atomiseur (26) selon l'une quelconque des revendications 1 à 6.

8. Dispositif de cigarette électronique (10) selon la revendication 7, **caractérisé en ce que** l'ouverture latérale (50) est voisine de l'élément de fixation (54).

9. Dispositif de cigarette électronique (10) selon la revendication 7 ou 8, **caractérisé en ce que** l'élément de fixation (54) comprend un filetage intérieur qui forme un bord de l'ouverture latérale (50).

10. Dispositif de cigarette électronique (10) selon l'une quelconque des revendications 7 à 9, **caractérisé en ce que** le dispositif de cigarette électronique (10) comprend un réservoir de liquide (34) pour du liquide devant être pulvérisé, et un volume de réception (56) pour l'atomiseur (26), le réservoir de liquide (34) étant voisin du volume de réception (56).

11. Dispositif de cigarette électronique (10) selon la revendication 10, **caractérisé en ce que** le réservoir de liquide (34) est formé avec une rainure (44), dans laquelle le volume de réception (56) est disposé, au moins par sections.

12. Dispositif de cigarette électronique (10) selon la revendication 10 ou 11, **caractérisé en ce que** le réservoir de liquide (34) comprend au moins une zone de sortie de liquide (A₃, A₄), la zone de sortie de liquide (A₃, A₄) étant perméable aux liquides.

13. Dispositif de cigarette électronique (10) selon la revendication 12, **caractérisé en ce que** le réservoir de liquide (34) comprend au moins deux zones de sortie de liquide (A₃, A₄) et un passage central (32) pour recevoir du liquide pulvérisé, le passage central (32) étant disposé entre les au moins deux zones de sortie de liquide (A₃, A₄).
